# EUROPEAN PATENT APPLICATION

(11) **EP 2 545 981 A2**
(43) Date of publication of application: **16.01.2013**
(21) Application number: 11753636.7
(22) Date of filing: 11.03.2011
(51) Int. Cl.: B01D 53/86, B01D 46/00, B01D 53/26

(54) **AIR-PURIFYING MODULE**

(30) Priority: 11.03.2010 KR 20100021949
(71) Applicant: E.M.W. Energy Co., Ltd., Geumcheon-gu, Seoul 153-803 (KR)
(72) Inventor: RYOU, Byung Hoon, Seoul 137-040 (KR); KONG, Jae Kyung, Seoul 137-790 (KR)
(74) Representative: Thoma, Michael
(86) International application number: PCT/KR2011/001705
(87) International publication number: WO 2011/112031

(57) **Abstract**

The present invention relates to an air-purifying module in which a heater heats a filter unit to purify air, wherein the filter unit includes an inorganic coating having a plurality of air pores, and a catalyst layer formed by impregnating a portion or the entirety of the inorganic coating with a catalyst mother liquid. According to one embodiment of the present invention, an air-purifying module comprises: an air-permeable filter unit; and a heater. An inorganic coating having a plurality of air pores is formed on the surface of the filter unit, and the filter unit has a catalyst layer formed by impregnating a portion or the entirety of the inorganic coating with a catalyst mother liquid, wherein the filter unit enables air to initiate a catalytic reaction with the catalyst layer under a predetermined temperature condition such that the air can be purified. The heater heats the filter unit such that the filter unit is maintained at said predetermined temperature condition

## Description

### TECHNICAL FIELD

The present invention relates to an air-purifying module, and more specifically, to an air-purifying module for purifying air by heating a filter unit using a heater, in which the filter unit includes an inorganic coating having a plurality of air pores and a catalyst layer formed by impregnating a portion or the entirety of the inorganic coating with a catalyst mother liquid.

### BACKGROUND ART

Generally, a filter unit for purifying exhaust gas is fabricated by coating a precious metal such as platinum or the like, which is a catalyst material for purifying the exhaust gas, on a carrier mainly formed of a ceramic material. However, the carrier formed of a ceramic material is vulnerable to impact and thus has low durability. In addition, weight of the carrier increases due to the nature of high density ceramic. The carrier formed of a ceramic material is difficult to mass produce since its manufacture cost is high.

In order to solve these problems, Korean Patent Application No. 2009-0036439 applied by the inventor of the present invention provides a carrier structure applicable to a gas reaction such as gas purification or the like and a method of fabricating a carrier, in which an inorganic membrane formed of porous inorganic coating is fabricated using an anodic oxidation reaction, and the inorganic coating is applied to the carrier.

However, since such a carrier structure operates through a catalytic reaction under a predetermined temperature condition, preferably 200 to 250°C, it will be referred to as a structure that is mainly used for purifying previously heated high temperature gas such as exhaust gas of a prime motor. That is, air of room temperature is difficult to purify with only a carrier structure of a conventional technique.

Accordingly, it is required to develop a durable, light-weighted and cheap air-purifying module capable of easily purifying and exhausting air of room temperature.

### DISCLOSURE OF INVENTION

### TECHNICAL PROBLEM

Therefore, one or more embodiments of the present invention has been made in view of the above problems, and it is an aspect of the present invention to provide an air-purifying module for purifying air by heating a filter unit using a heater, in which the filter unit includes an inorganic coating having a plurality of air pores and a catalyst layer formed by impregnating a portion or the entirety of the inorganic coating with a catalyst mother liquid.

### TECHNICAL SOLUTION

To accomplish the above aspect, according to an embodiment of the present invention, there is provided an air-purifying module including: an air-permeable filter unit including an inorganic coating and a catalyst layer, in which the inorganic coating having a plurality of air pores is formed on a surface of the filter unit, and the catalyst layer is formed by impregnating a portion or an entirety of the inorganic coating with a catalyst mother liquid, the filter unit for purifying air by triggering a catalytic reaction with the catalyst layer under a predetermined temperature condition; and a heater for heating the filter unit under the predetermined temperature condition.

The air-purifying module according to an embodiment of the present invention further includes a cooling heat exchanger for cooling the air heated while passing through the filter unit.

The air-purifying module according to an embodiment of the present invention further includes a heating heat exchanger for heating the air before passing through the filter unit.

The air-purifying module according to an embodiment of the present invention further includes a cooling and heating heat exchanger including a plurality of first slots formed to penetrate in a vertical direction and a plurality of second slots formed to penetrate in a horizontal direction between the first slots respectively, in which either of the first and second slots passes the air before passing through the filter unit and the other slot passes heated air after passing through the filter unit to accomplish heat exchange so that the air before passing through the filter unit is heated up and the heated air after passing through the filter unit is cooled down.

The inorganic coating is formed through an anodic oxidation phenomenon.

The catalyst layer is a catalyst layer of platinum (Pt) or rhodium (Rh).

The predetermined temperature condition is 200 to 250°C.

The filter unit is formed by stacking a plurality of plates to be spaced apart from each other so that the air may flow between the plates.

A plurality of air vent holes may be formed on the plate.

The heater may be formed in a shape of a bar, which is combined with the plates while penetrating the plates.

The heater may be formed to be spaced apart from a center of the plate by a predetermined distance toward an air flow-in direction and to penetrate and be bonded to the plates.

The air-purifying module may further include an insulator for blocking the heat generated by the heater so that the heat may not be transferred to constitutional components other than the filter unit.

### ADVANTAGEOUS EFFECTS

The air-purifying module according to one or more embodiments of the present invention is durable and light-weighted compared with a ceramic carrier.

In addition, according to one or more embodiments of the air-purifying module of the present invention, since an effect of purifying air as high as or further superior to that of existing ceramic carriages can be obtained although only a small amount of catalyst is used, manufacturing cost may be reduced.

In addition, since one or more embodiments of the air-purifying module of the present invention configure a carrier using a material of high heat conductivity, even air of room temperature can be effectively purified.

### BRIEF DESCRIPTION OF THE DRAWINGS

FIG. 1 is a cross-sectional view showing an inorganic coating having a plurality of air pores formed on the surface of a filter unit and a catalyst layer formed by impregnating the inorganic coating with a catalyst mother liquid.

FIG. 2 is a block diagram showing an air-purifying module according to an embodiment of the present invention.

FIG. 3 is a block diagram showing an air-purifying module according to another embodiment of the present invention.

FIG. 4 is a block diagram showing an air-purifying module according to still another embodiment of the present invention.

FIG. 5 is a block diagram showing an air-purifying module according to still another embodiment of the present invention.

FIG. 6 is a perspective view and a partially enlarged view showing the structure of a cooling and heating heat exchanger of an air-purifying module according to an embodiment of the present invention.

FIG. 7 is a perspective view showing a filter unit combined with a heater in an air-purifying module according to an embodiment of the present invention.

FIG. 8 is a cross-sectional view showing an air-purifying module according to an embodiment of the present invention.

### DETAILED DESCRIPTION

The preferred embodiments of the present invention will be hereafter described in detail, with reference to the accompanying drawings. Furthermore, in the drawings illustrating the embodiments of the present invention, elements having like functions will be denoted by like reference numerals and details thereon will not be repeated.

Generally, a structure impregnating and supporting a catalyst is referred to as a carrier, and a carrier of a ceramic material may be used. Instead of a carrier of a ceramic material, a carrier of a metallic material formed with an inorganic coating having a plurality of air pores on the surface can be used, and in the embodiments of the present invention, a catalyst layer formed by impregnating such a carrier with a catalyst mother liquid will be used as a filter unit. The inorganic coating having a plurality of air pores on the surface of a carrier of a metallic material can be formed through an anodic oxidation reaction.

Anodic oxidation is an oxidation phenomenon occurring when an anodic reaction occurs, and if the anodic oxidation is used, a process of developing an oxide or nitride film formed on a metallic surface can be performed using an electrolytic reaction.

When such anodic oxidation occurs, a microscopic change may occur on the metallic surface, or a change may occur in the crystal structure of the metal, and an example of the anodic oxidation is as described below.

If direct current flows through electrolyte solution, hydrogen is generated at the cathode metal, and oxygen is generated at the anode metal (e.g., an aluminum (Al) alloy, titanium (Ti), zinc (Zn), magnesium (Mg), niobium (Nb), etc). The oxygen generated at this point reacts with the anode metal and forms a metallic oxide film, and the electrolyte solution minutely dissolves the formed oxide film. If the dissolving speed keeps a balance with the speed of forming the oxide film, a plurality of air pores having a diameter of 10 to 150nm is formed on the surface of the anode metal.

When the air pores are formed, the electrolyte solution and the current may contact with a metal matrix existing at the bottom of the oxide film, and as a result, a film much thicker than the oxide film formed by the spontaneous oxidation reaction of the metal may be formed.

The film formed through such a process has various physical properties depending on the condition of the process, and if an electrolyte solution of a lower concentration and current and/or voltage of a higher magnitude are used, a thicker film is formed.

The oxide film formed in the method described above can be used as an inorganic coating of the air-purifying module according to an embodiment of the present invention. If the inorganic coating formed as described above is used, an air-purifying module of a low price and a high performance can be fabricated.

The inorganic coating can be fabricated using a conductive metal, and aluminum can be used as an example of the conductive metal. If an anodic oxidation reaction is triggered using the aluminum as an anode, alumina, i.e., an aluminum oxide, is stacked slowly, and an alumina film formed as such is used as the inorganic coating of an embodiment of the present invention.

Next, a catalyst layer of platinum (Pt) or rhodium (Rh) may be inserted between the air pores of the inorganic coating. The catalyst layer is formed by impregnating a catalyst mother liquid, and when catalyst mother liquid is dried, fabrication of the catalyst layer is completed.

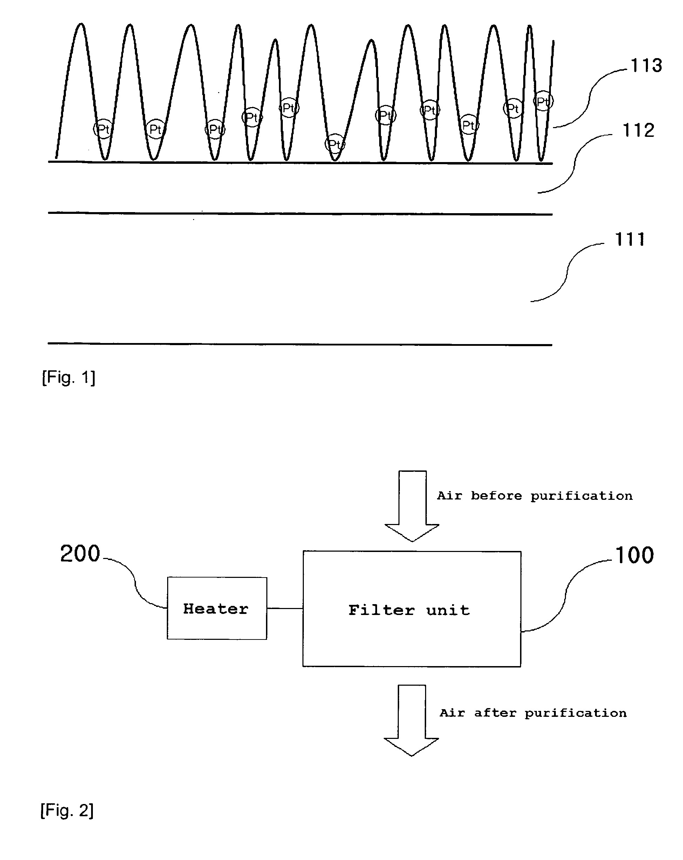

FIG. 1 shows the cross section of a structure including a metallic layer 111 functioning as a base of a filter unit, a transition layer 112 where a metal configuring the metallic layer 111 and an oxide of the metal coexist on the metallic layer 111, and an inorganic coating 113 formed on the transition layer 112. It is shown in FIG. 1 that platinum Pt is formed in a plurality of air pores contained in the inorganic coating 113, as an example of the catalyst layer.

A part which actually contributes to the chemical reaction for purifying air is the catalyst layer, and since a method of forming a catalyst layer by impregnating a carrier with a catalyst mother liquid obtains an effect of extending a surface area, it is advantageous compared with a method of using only a metal forming the catalyst layer as a filter. In addition, since a metal such as the platinum Pt forming the catalyst layer is expensive, it is advantageous from the aspect of cost.

Hereinafter, the structure of an air-purifying module according to a variety of embodiments of the present invention will be described in detail.

Referring to FIG. 2, the most fundamental form of the air-purifying module according to an embodiment of the present invention includes a filter unit 100 and a heater 200 for heating the filter unit 100. The filter unit 100 is a structure constructed to allow flow of air, and the structure allowing flow of air is a structure through which a gas may pass. That is, it should be a structure through which particles in the air to be purified may pass while colliding with the surface of the filter unit 100.

The filter unit 100 is formed by stacking a plurality of plates 120 to be spaced apart from each other so that the air may flow between the plates 120. Alternatively, the filter unit 120 may be formed in the shape of a barrel such as a cylinder or the like so that gas may pass through the inner space of the barrel. Alternatively, the filter unit 120 may be rolled in the shape of a spiral so that the air may pass through. As described above, the filter unit 100 may be formed in a variety of structures, and it should be understood that any filter configured to flow air represents the technical spirits of the present invention regardless of the shape.

An inorganic coating having a plurality of air pores is formed on the surface of the filter unit 100, and a catalyst layer is formed by impregnating a portion or the entirety of the inorganic coating with a catalyst mother liquid. These can be formed by performing an anodic oxidation reaction as described above.

In the air-purifying module including the filter unit 100 and the heater 200 for heating the filter unit 100, the heater 200 heats the filter unit 100 to maintain a temperature, for example, between 200 and 250°C so that the air passing through the filter unit 100 may perform a catalytic reaction. While the filter unit 100 is heated, volatile organic materials or environmental hormones such as formaldehyde are changed into carbon dioxide and water harmless to a human body by the catalytic reaction. In addition, biochemical contaminants such as fungi, spores and the like are chemically burnt and removed by the catalytic reaction. Furthermore, toxic substances such as carbon monoxide, nitrogen monoxide and the like are changed into carbon dioxide, nitrogen and water by the catalytic reaction.

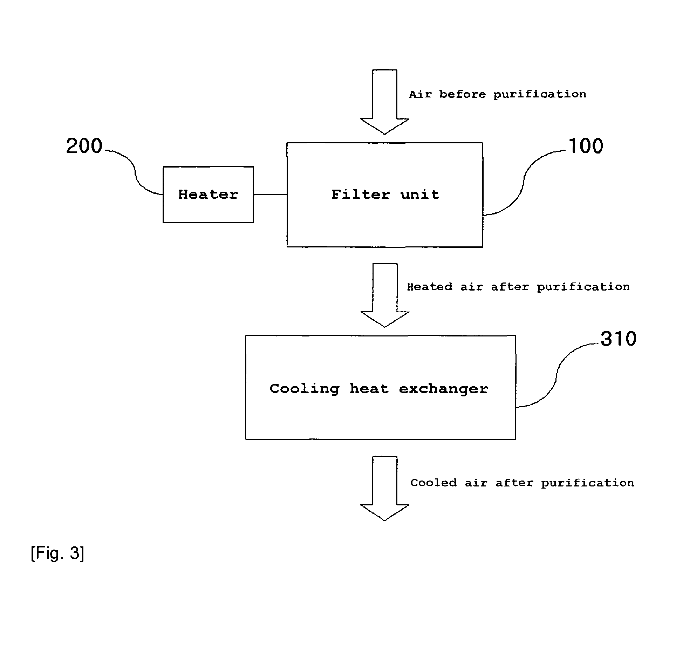

Referring to FIG. 3, the air-purifying module according to an embodiment of the present invention may further include a cooling heat exchanger 310. Air of high temperature passing through the filter unit 100 may be cooled down and exhausted outside the air-purifying module by the cooling heat exchanger 310. The cooling heat exchanger 310 may have a structure capable of cooling the high temperature air in a method of cooling air using outer air of room temperature.

Since users of an apparatus mounted with the air-purifying module may feel exhaustion of the air cooled down close to the room temperature through the cooling heat exchanger 310, satisfaction of the users in using the apparatus may be enhanced.

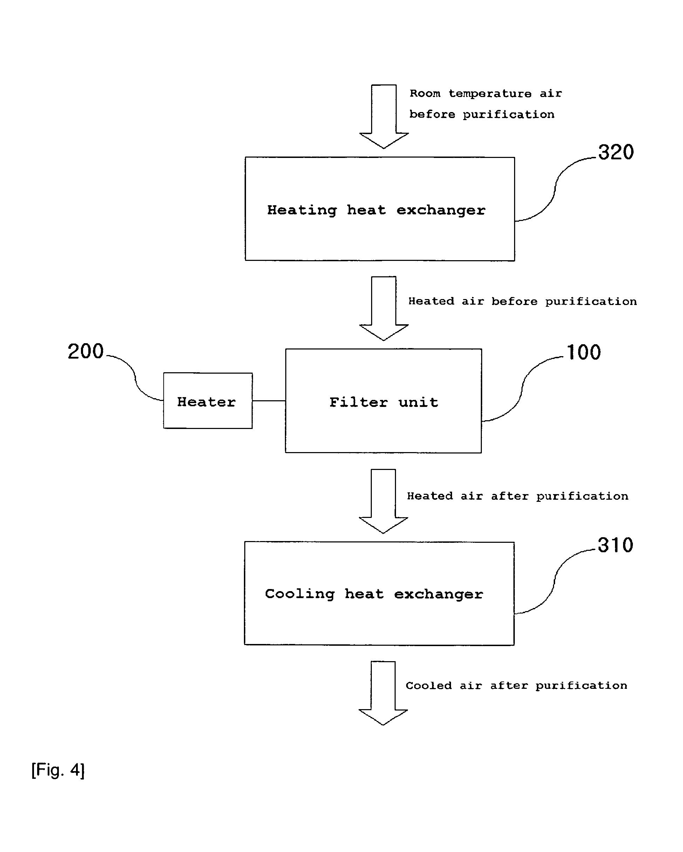

Referring to FIG. 4, the air-purifying module according to an embodiment of the present invention may further include a heating heat exchanger 320. The heating heat exchanger 320 helps facilitating the catalytic reaction by heating air before the air passes through the filter unit 100. That is, since efficiency of the catalytic reaction may be lowered if the air maintains room temperature until right before arriving at the filter unit 100 and starts to be heated right after arriving at the filter unit 100, the efficiency of the catalytic reaction can be enhanced by heating the air in advance before the air arrives at the filter unit 100.

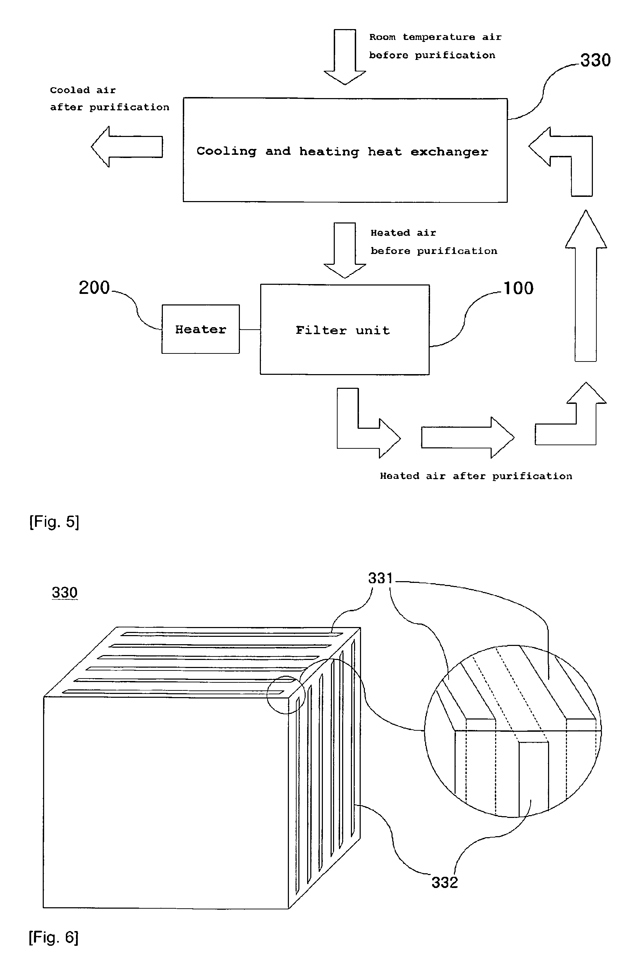

Referring to FIG. 5, the air-purifying module according to an embodiment of the present invention may include a cooling and heating heat exchanger 330 for simultaneously performing both functions of the cooling heat exchanger 310 and the heating heat exchanger 320. That is, the air-purifying module may include the filter unit 100, the heater 200 and the cooling and heating heat exchanger 330.

Referring to FIG. 6, the cooling and heating heat exchanger 330 includes a plurality of first slots 331 formed to penetrate in the vertical direction and a plurality of second slots 332 formed to penetrate in the horizontal direction between the first slots 331 respectively. Either of the first and second slots 331 and 332 cools down the air after the air passes through the filter unit 100, and the other slot heats the air before the air passes through the filter unit 100.

For example, if the air passing through the second slot 332 is high temperature air and the air passing through the first slot 331 is room temperature air, the air passing through the second slot 332 transfers heat to the cooling and heating heat exchanger 330, and the transferred heat is delivered to the room temperature air passing through the first slot 331. Accordingly, the air passing through the first slot 331 has an effect of being heated up, and the air passing through the second slot 332 has an effect of being cooled down.

Meanwhile, the expression of vertical or horizontal direction does not mean a vertical direction or a horizontal direction with respect to an absolute reference, but expresses a relative reference with respect to each of the vertical and horizontal directions, and they should not be construed as limiting the technical spirits intended by the present invention.

In addition, although the first slot 331 is used for heating and the second slot 332 is used for cooling in FIG. 5, it makes no difference to use the first slot 331 for cooling and the second slot 332 for heating in reverse.

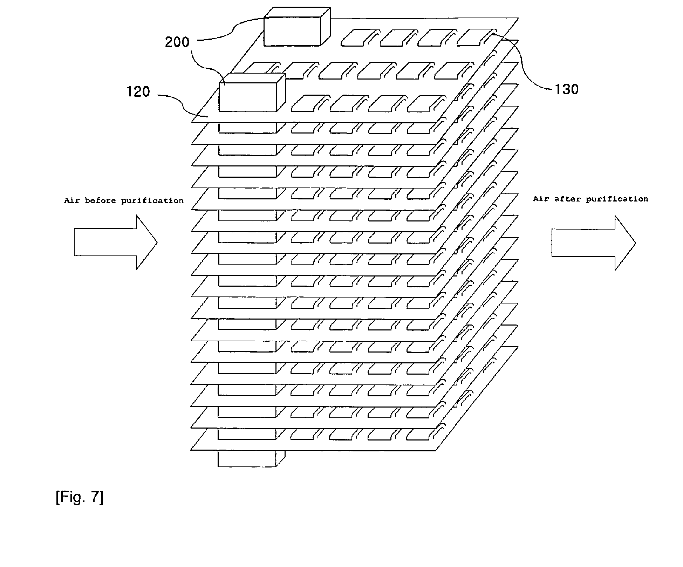

Referring to FIG. 7, the filter unit 100 of the air-purifying module according to an embodiment of the present invention has a plurality of plates 120 formed to be stacked and spaced apart from each other, and thus the filter unit 100 is configured to flow air between the plates 120.

At this point, a plurality of air vent holes 130 may be formed on the plate 120. If some of the air gets out through the air vent holes 130 and passes by a plurality of plates 120, rather than all the air passes only through the spaces formed between the plates 120, an effect of extending a contact area can be obtained, and thus the catalysis reaction may be occurred further efficiently.

As shown in FIG. 7, the heater 200 is formed in a shape of a bar and bonded to the plates 120 while penetrating the plates 120. A positive temperature coefficient (PTC) heater may be used as the heater 200, and the number of the heaters 200 may be adjusted depending on the area or number of the plates 120.

The heater 200 may be formed to be spaced apart from the center of the plate 120 by a predetermined distance toward the air flow-in direction and to penetrate and be bonded to the plates 120. There is a problem in that since temperature of the inflow air is low compared with that of the filter unit 120, the inflow air cools down the plates 120, and temperature of the plates 120 is not uniform. A certain temperature condition, for example, 200 to 250°C, should be maintained in order to perform a catalytic reaction. However, since temperature of the plate 120 is comparatively low at a position where the air flows in, efficiency of the catalytic reaction can be lowered. Accordingly, the heater 200 is combined at a position where the air flows in, and thus the inflow air is heated first, and the overall efficiency of the catalytic reaction is improved thereby.

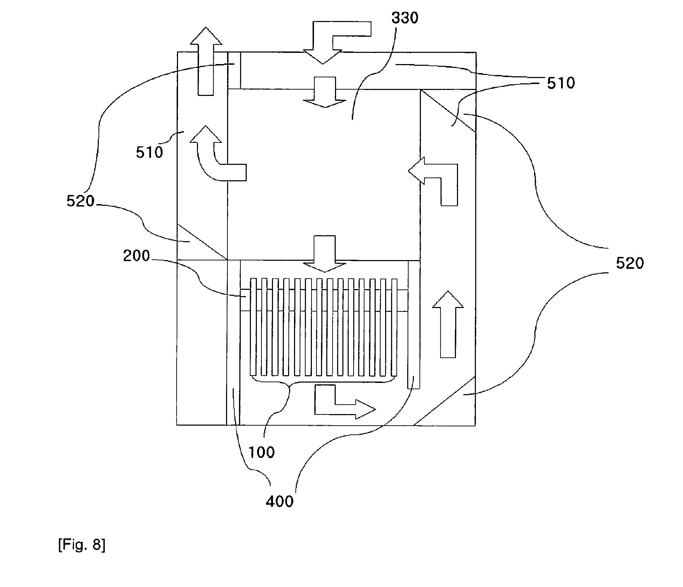

Referring to FIG. 8, the air-purifying module according to an embodiment of the present invention further includes an insulator 400 for blocking the heat generated by the heater 200 so that the heat may not be transferred to the constitutional components other than the filter unit 100. The passage unit 510 in FIG. 8 is an empty space which functions as a passage through which air flows. An air flow induction unit 520 is a portion that is blocked so that the air may not pass through, and it induces a direction in which the air flows. The arrow symbols show flow of the air. Air of room temperature before purification flowing into the air-purifying module from the top is heated while passing through the cooling and heating heat exchanger 330 in the vertical direction and is purified while passing through the filter unit 100 heated by the heater 200. After being purified, the heated air flows toward lower right and then upward and is cooled down while passing through the cooling and heating heat exchanger 330 in the horizontal direction. The cooled air flows out of the air-purifying module through the left top side. However, the reference directions described above merely indicate the directions shown in FIG. 8, and the installation direction of the air-purifying module may be a relative direction.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

### INDUSTRIAL APPLICABILITY

Although the air-purifying module according to the present invention can be used to be mounted on an air purifier apparatus, the air-purifying module may be applied to a variety of devices such as an air conditioner, a fan heater or the like and used as a part for purifying air.

## Claims

1. An air-purifying module comprising:
an air-permeable filter unit including an inorganic coating and a catalyst layer, in which the inorganic coating having a plurality of air pores is formed on a surface of the filter unit, and the catalyst layer is formed by impregnating a portion or an entirety of the inorganic coating with a catalyst mother liquid, the filter unit for purifying air by triggering a catalytic reaction with the catalyst layer under a predetermined temperature condition; and
a heater for heating the filter unit under the predetermined temperature condition.

2. The module according to claim 1, further comprising a cooling heat exchanger for cooling the air heated while passing through the filter unit.

3. The module according to claim 2, further comprising a heating heat exchanger for heating the air before passing through the filter unit.

4. The module according to claim 1, further comprising a cooling and heating heat exchanger including a plurality of first slots formed to penetrate in a vertical direction and a plurality of second slots formed to penetrate in a horizontal direction between the first slots respectively, wherein
either of the first and second slots passes the air before passing through the filter unit and the other slot passes heated air after passing through the filter unit to accomplish heat exchange so that the air before passing through the filter unit is heated up and the heated air after passing through the filter unit is cooled down.

5. The module according to any one of claims 1 to 4, wherein the inorganic coating is formed through an anodic oxidation phenomenon.

6. The module according to any one of claims 1 to 4, wherein the catalyst layer is a catalyst layer of platinum (Pt) or rhodium (Rh).

7. The module according to any one of claims 1 to 4, wherein the predetermined temperature condition is 200 to 250°C.

8. The module according to any one of claims 1 to 4, wherein the filter unit is formed by stacking a plurality of plates to be spaced apart from each other so that the air may flow between the plates.

9. The module according to claim 8, wherein a plurality of air vent holes is formed on the plate.

10. The module according to claim 8, wherein the heater is formed in a shape of a bar, which is bonded to the plates while penetrating the plates.

11. The module according to claim 10, wherein the heater is formed to be spaced apart from a center of the plate by a predetermined distance toward an air flow-in direction and to penetrate and be bonded to the plates.

12. The module according to any one of claims 1 to 4, further comprising an insulator for blocking the heat generated by the heater so that the heat may not be transferred to constitutional components other than the filter unit.
